# EUROPEAN PATENT APPLICATION

(11) **EP 2 505 124 A2**
(43) Date of publication of application: **03.10.2012**
(21) Application number: 10833519.1
(22) Date of filing: 22.11.2010
(51) Int. Cl.: A61B 1/24, A61B 1/06

(54) **LED ORAL ILLUMINATOR USING A BITE BLOCK**

(30) Priority: 24.11.2009 KR 20090113654; 27.08.2010 KR 20100083313
(71) Applicant: Dentozone Co., Ltd., Geumcheon-Gu, Seoul 153-801 (KR)
(72) Inventor: KOO, Cha Hyoung, Seoul 153-013 (KR)
(74) Representative: Krauns, Christian
(86) International application number: PCT/KR2010/008227
(87) International publication number: WO 2011/065707

(57) **Abstract**

Disclosed herein are intraoral illumination devices. More specifically, the LED intraoral illumination device in accordance with the present invention has an improved battery for providing power supply and structure of a bit block for opening a mouth using the bit block.

## Description

### [Technical Field]

The present invention relates to intraoral illumination devices, and more particularly to an LED oral illuminator having a battery for providing power supply and an improved structure of a bit block for opening a mouth using the bit block.

### [Background Art]

In general, patients lie in dental unit chair in performing dental treatment. An external light source using illumination device of the dental unit chair illuminate the mouth cavity of a patient to enable a doctor to easily and clearly observe the mouth cavity.

Then, a doctor illuminates lesion of the mouth cavity of a patient using three-way syringe, high speed handpiece, scaler, and low speed handpiece and simultaneously performs dental treatment.

Meanwhile, the size of lesion in the mouth cavity a patient is too small and connected with a lot of nerves. For this reason, detail treatment and remedy are required. In specifically, it is very important for a doctor to clearly observe the mouth cavity of a patient.

A conventional intraoral illumination device, however, should be manipulated in performing dental treatment. Thus, it is difficult for skilled doctor or nurse to clearly illuminate the mouth cavity of a patient. Also, when the mouth cavity of a patient is illuminated using illumination device of dental unit chair, there is a problem that the mouth cavity of a patient is not illuminated as a whole. The reason is that the mouth cavity of a patient is illuminated from outside the mouth cavity.

In addition, the conventional intraoral illumination device is connected with the dental unit chair through power wire, thereby causing inconvenience to doctor and patient when dental treatment is performed.

In order to improve these problems, the present applicant invented an intraoral illumination device with mouth gag, which is disclosed in Korea Patent No. 10-0673479. This patent discloses that both lips of a patient is kept opening using a mouth gag, and battery is embedded in the intraoral illumination device to illuminate the mouth cavity.

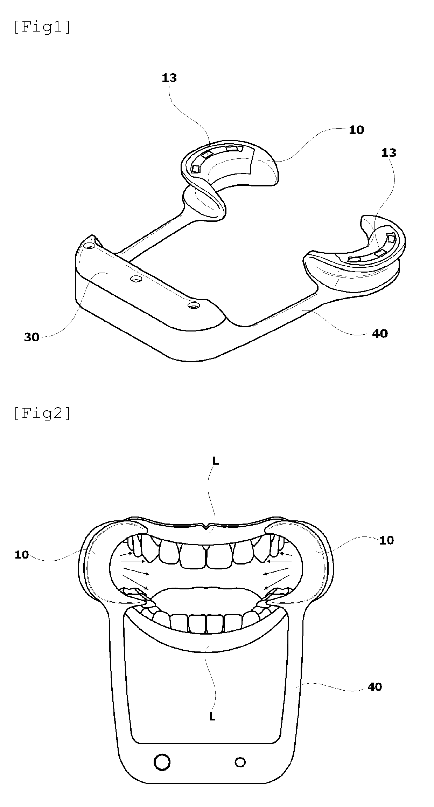

Referring to FIGS. 1 and 2, the intraoral illumination device with the mouth gag is described in more detail below. The intraoral illumination device with the mouth gag comprises a couple of holders (10, mouth gag holder), a battery case 30, and a body 40.

The couple of holders 10 include an LED light source 13 and are facing to each other with a predetermined interval. With both lips of a patient opened and supported, the mouth cavity of a patient is illuminated using the LED light source 13.

In other words, since the mouth cavity can be illuminated with the mouth opened by holding both lips employing the mouth gag holder 10, there is an advantage that additional illumination device is not necessary.

However, these methods have their own problems. Because the illumination device holds both lips of a patient, it is difficult for a doctor to smoothly manipulate upper and lower teeth when dental treatment is performed. Also, the usage of handpiece and scaler is interrupted. This causes difficulty in subsequent processes.

The holder 10 and the LED light source are equipped in one entity. Accordingly, in the event that the intraoral illumination device is reused after exposing it at high temperature steam to be sterilized, there is a high possibility that the LED light source 13 is damaged or the life thereof is shortened owing to the high temperature steam.

Further, in the conventional intraoral illumination device, since the mouth of a patient is opened by supporting lips and presses gingiva, thereby bringing about pain.

In addition, the lips of a patient are disposed and supported by the holder 10, and thus some patients can encounter problems in their lips to use the holder due to his/her mouth size.

Recently, the present applicant has proposed intraoral illumination device and surgical illumination device in mouth cavity using a bite block capable of illuminating which is disclosed in Korea Patent Nos. 10-2009-0025977 and 10-2009-0031645. According to the disclosed inventions, the intraoral illumination device can illuminate mouth cavity and occiput of a patient after opening a mouth by naturally biting a bite block by patient's teeth without supporting lips through the holder 10.

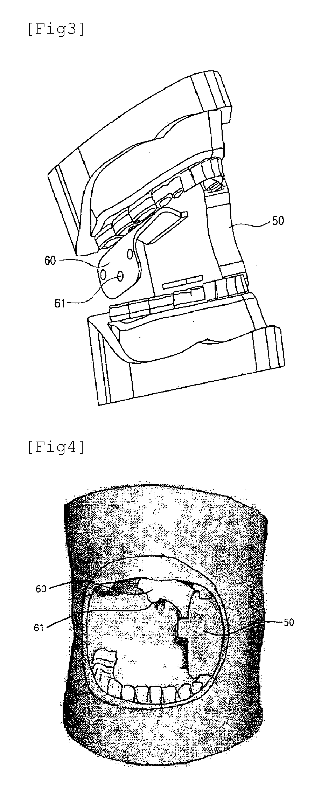

Referring to FIGS. 3 and 4, the intraoral illumination device and surgical illumination device in mouth cavity using the bite block comprises a bite block bitten between teeth 50 and an illumination plate 60 extended from the bite block 50 to have several lightening 61.

Since dental treatment is performed under the condition that a patient naturally bites the bite block 50 between upper and lower teeth in one side of a mouth, a patient suffering from jaw pain can use without unwelcome attention. The illumination plate 60 is separated from the bite block 50, so that the bite block 50 can be washed sanitarily.

The intraoral illumination device and surgical illumination device embeds a battery, they are not disposable. Also, the bite block 50 cannot be washed in itself, so that it is difficult to be kept clean.

The lightening 61 is mounted on the illumination plate 60 extended from the bite block 50, thereby generating the formation of shadow by the illumination plate 60. In specifically, it is not easy to illuminate lingual sides of upper and lower teeth, the other ends of the upper and lower teeth facing moral biting the bite block, throat, subglossal and so forth.

The intraoral illumination device and surgical illumination device are positioned in the mouth of a patient. There is a problem that a patient cannot easily close his/her lips. Adopting additional aid is inconvenient when opening the mouth of the patient.

In the event that the battery is discharged when dental treatment is performed, this method can be inconvenient in swapping the batteries, and when the battery is charged after extracting the intraoral illumination device and the surgical illumination device outside the mouth of the patient.

### [Disclosure]

### [Technical Problem]

The inventors of the present invention completed the present invention resulting from efforts to develop an LED oral illuminator using a bite block which is capable of practicing a patient without extracting the LED oral illuminator to the outside of a mouth of the patient when it is discharged, and can enable a doctor to easily observe a mouth cavity, in particularly, lingual sides of upper and lower teeth, the other ends of the upper and lower teeth facing moral biting the bite block, throat, subglossal by clearly illuminating them as a whole, and cannot cause inconvenience of a patient by reliably opening a mouth of a patient, and can be effectively washed to be maintained sanitarily.

Accordingly, an object of the present invention is to provide an LED oral illuminator using a bite block which is capable of enabling a doctor to easily observe a mouth cavity, in particularly, lingual sides of upper and lower teeth, the other ends of the upper and lower teeth facing moral biting the bite block, throat, subglossal by effectively illuminating them.

Another object of the present invention is to provide an LED oral illuminator using a bite block in which the bite block can be disposable and maintained in sanitary due to its washability in a high temperature when it is reused.

A further object of the present invention is to provide an LED oral illuminator using a bit block that can allow a mouth of a patient to be comfortably opened when dental treatment is performed.

Yet another object of the present invention is to provide an LED oral illuminator using a bite block in which a battery of the bite block can be easily charged or swapped in the event that the battery is discharged while dental treatment is performing.

The objects of the invention are not limited to the above-mentioned objects, and other unmentioned objects will be clearly interpreted by those skilled in the art from the following description.

### [Technical Solution]

Embodiments of the present invention provide an LED oral illuminator using a bite block comprising a frame being bent to be U-shaped as a whole and a bite block coupled to one end of the frame. The frame comprises an LED light source mounted on one end of the frame and a light source control switch for controlling turn on/off of the LED light source.

In some embodiments of the present invention, a battery mounted on the other end of the frame is further included.

In other embodiments of the present invention, the LED light source comprises a plurality of LED lamps.

In further embodiments of the present invention, the bit block comprises a block body bitten between upper and lower teeth in one side of a mouth of a patient to open the mouth of the patient and a light source insert hole formed in one side of the block body so that the LED light source us inserted into the light source insert hole.

In other embodiment of the present invention, a bite groove bitten between the upper and lower teeth is formed at upper and lower portions of the block body.

In yet other embodiment of the present invention, the bite block is made of transparent material so as to transmit the light source into the mouth.

In further embodiment of the present invention, the battery is chargeable, and the frame further includes an electrical charging terminal for charging the battery.

In other embodiment of the present invention, the frame further includes brightness controller for adjusting brightness of the LED light source.

In further embodiment of the present invention, the LED light source further includes an UV LED lamp for emitting ultraviolet rays.

### [Advantageous Effects]

The present invention has the following effects.

First, an LED oral illuminator using a bite block according to the present invention enables a doctor to effectively observe a mouth cavity, in particularly, lingual sides of upper and lower teeth, the other ends of the upper and lower teeth facing moral biting the bite block, throat, subglossal by clearly illuminating them as a whole because of employing an LED light source located in the bite block.

Furthermore, in the present invention, a frame including the bite block and the LED light source can be separated from each other and can be attachable, so that the bite block can be disposable. When the bite block is reused, it can be sterilized in high temperature to be kept clean. Thereby, it is possible to protect secondary infection of patients.

In addition, in the present invention, a frame located outside from a mouth of a patient bent to be U-shaped is coupled to a bite block in the mouth of the patient. For this reason, one side of lip of the patients can be supported by these frame, it is possible for the patient to continuously open the mouth without additional efforts. Also, this can remove the apprehension of a patient suffering from jaw pain.

Moreover, in the present invention, the battery is equipped in the frame, that is, the outside of a mouth of a patient. As a result, even though the battery is discharged in performing dental treatment, the battery can be chargeable and swapped without extracting it to the outside of the mouth of the patient, thereby continuously performing dental treatment. By increasing a capacity of the battery, the illumination time can be improved.

### [Description of Drawings]

FIG. 1 is a perspective view showing a conventional intraoral illumination device with a mouth gag;
FIG. 2 is a perspective view showing installation of a conventional intraoral illumination device with a mouth gag in a mouth of a patient;
FIG. 3 is a perspective view showing an illumination device in a mouth cavity using a conventional bite block that can illuminate a mouth of a patient;
FIG. 4 is a perspective view showing a conventional surgical illumination device in a mouth cavity;
FIG. 5 is a perspective view showing a LED oral illuminator in accordance with an embodiment of the present invention;
FIG. 6 is an exploded perspective view showing a frame and a bite block of a LED oral illuminator in accordance with an embodiment of the present invention; and
FIG. 7 is a perspective view showing installation of a LED oral illuminator in accordance with an embodiment of the present invention.

Reference should now be made to the drawings, in which the same reference numerals are used throughout the different drawings to designate the same or similar components.

### <Brief explanation of essential parts of the drawings>

- 100:: Mouth Cavity
- 200:: Bite Block
- 210:: Block Body
- 211:: Light Source Insert Hole
- 214:: Bite Groove
- 215:: Bite Projection
- 300:: Frame
- 310:: Electrical Charging Terminal
- 320:: Light Source Control Switch
- 330:: Battery
- 340:: LED Light Source

### [Best Mode]

The terminology which is used in common will be used for the purpose of description and not of limitation. Furthermore, terms and words used by the applicant may be used for special cases. In this case, the meaning of terms or words must be understood wit due regard to the meaning expressed in the specification rather than taking into account only the basic meaning of the terms and words.

Hereinafter, the technical construction of the present invention will be described in detail with reference to preferred embodiments illustrated in the attached drawings.

The present invention may, however, be embodied in different forms and should not be construed as limited to the embodiments set forth herein. The same reference numeral is used to refer to like elements throughout.

As used herein, the terms "about", "substantially", etc. are intended to allow some leeway in mathematical exactness to account for tolerances that are acceptable in the trade and to prevent any unconscientious violator from unduly taking advantage of the disclosure in which exact or absolute numerical values are given so as to help understand the invention.

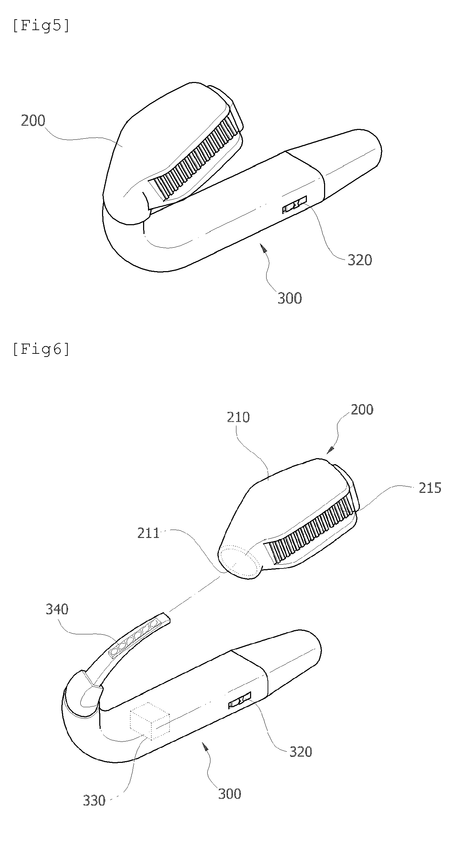

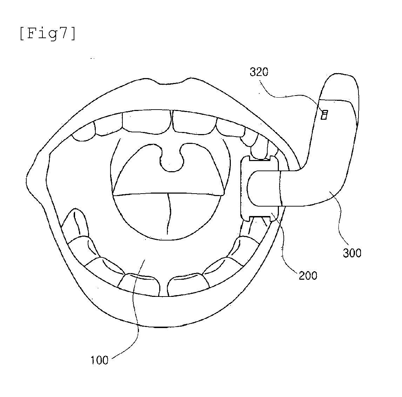

FIG. 5 is a perspective view showing a LED oral illuminator in accordance with an embodiment of the present invention. FIG. 6 is an exploded perspective view showing a frame and a bite block of a LED oral illuminator in accordance with an embodiment of the present invention. FIG. 7 is a perspective view showing installation of a LED oral illuminator in accordance with an embodiment of the present invention.

With reference to FIGS. 5 to 7, an LED oral illuminator using a bite block according to an embodiment of the present invention a frame 300 included outside a mouth 100 and a bite block 200 included in the mouth and bitten between upper and lower teeth in one side of the mouth.

The frame 300 is bent to be U-shaped. One end of the frame 300 is inserted into the mouth of a patient, and the other end of the frame 300 is exposed to the outside of the mouth of the patient to naturally cover lip and cheek in one side of the patient. The edge lip of the patient is supported by the bent-up of the U-shaped frame 300. Thus, the mouth of the patient can be kept opening comfortable and safe.

Additionally, the frame 300 comprises an LED light source 340, a light source control switch 320, a battery 330, and an electrical charging terminal 310.

Also, the frame 300 may further comprise a brightness controller (not shown) for adjusting brightness of an LED light source 340, which will be described later. The brightness controller adjusts brightness by controlling power supply provided to the LED light source 340.

The LED light source 340 is mounted on one end of the frame 300 to be inserted into the mouth of the patient. The LED light source 340 includes a plurality of LED lamps to illuminate a mouth cavity of the patient. The LED light source 340 is turned on/off when it is housed in the bite block 200 that will be descried later.

The LED light source 340 is extended from one end of the frame 340 to be a bar-shaped light unit. Accordingly, the LED light source can effectively illuminate deep portions in the mouth of the patient such as throat, subglossal and so forth. Comparing with an intraoral illumination device using a conventional bite block and surgical illumination device in a mouth, there is an advantage that lingual side of upper and lower teeth that is difficult to illuminate as well as upper and lower teeth in other side of a mouth facing a moral biting the bite block can be illuminated.

In order to prevent bacteria infection which may be caused when treatment is performed, sterilize bacteria, or distinguish dental caries, the LED light source 340 may comprise an UV LED lamp for emitting ultraviolet rays. The LED lamp and a plurality of lamps may be included simultaneously.

In other words, the LED lamps as well as certain lamps that are small and can illuminate a mouth cavity of human being are generally applicable as the LED light source 340.

The light source control switch 320 is positioned at an external surface of the frame 300 to control turn on/off of the LED light source 340. In an embodiment of the present invention, the light source control switch 320 employing a touched-type for a patient to easily turn on/off is described. In general, however, a switch with various kinds of types such as button-type or contact-type can be adopted.

The battery 330 is received in the other end of the frame 300 to be mounted. The battery 330 provides power supply to the LED light source 340, and more preferably, is chargeable.

That is, the battery 330 is not located in a mouth of a patient but outside the mouth of the patient. Since the size of the battery 330 is not relatively limited, the capacity of the battery 330 can be large.

The battery 330 includes a cell, a protection circuit, and a current counter, which are not shown.

The cell performs a function to charge and discharge stored power supply and provides charged power supply to the LED light source 340.

The protection circuit prevents cell damage by over current and over voltage when the cell is charged with external power supply.

The current counter counts the amount of current that inflows or outflows to the cell through a charging terminal 310 that will be described later and then, transfers enumerated value to a control unit (not shown). The control unit transmits a signal by the enumerated value to a displaying unit (not shown) such as charging lamps or LCD to display charging or discharging state of the battery 330.

The charging terminal 310 is a terminal for charging the battery 330 with external power supply and includes a socket electrically connected to external power supply.

The charging terminal 310 can charge the battery 330 even when the battery 330 provides power supply to the LED light source 340. Therefore, when the battery 330 is discharged in performing dental treatment, it can be charged without extracting it to the outside of a mouth of a patient.

The bite block 200 is removal coupled to the frame 300 and can be coupled to one end of the frame 300. The bite block 200 is bitten between upper and lower teeth in the mouth of the patient and supports the mouth opened. The bite block 200 includes a block body 210, a light source insert hole 211, a bite groove 214, and a bite projection 215.

The bite block 200 may be separated from the frame 300, so that it is disposable and replaceable. When the bite block 200 is reused, it can be sterilized in high temperature to be kept clean.

In order for the bite block 200 to be swapped and used depending on a mouth state or mouth size of patients, the bite block 200 may be a set of bite block including a plurality of blocks that have different size.

The block body 210 is bitten between the upper and lower teeth in the mouth of the patient to open the mouth and included in a body of the bite block 200.

The block body 210 is made of resin material that is harmless to human. Harmless materials like latex, rubber, silicone and so forth may be coated on an external surface of the block body.

The LED light source 340 houses the block body 210 to illuminate light into the mouth of the patient. The block body 210 may comprise a transparent block body allowing light emitted from the LED light source to be transmitted into the mouth of the patient.

Accordingly, a blind spot being a region of a mouth of a patient not illuminated by LED light source 340 is removed, and at the same time the formation of shadow is not created while illuminating the mouth cavity. Resultantly, a doctor can practice a mouth cavity of a patient in detail.

The light source insert hole 211 is formed at one side of the block body 200 and inserted into the LED light source 340.

The light source insert hole 211 is formed in order for light of the LED light source inserted into the bite block 200 to illuminate a center of a mouth cavity of the patient.

The bite groove 214 is included on upper and lower portions of the block body 210. The bite groove 214 has U-shaped cross-section to be bitten between the upper and lower teeth of the patient. The bite block 200 is not separated from teeth of the patients without additional efforts, thereby effectively fixing the bite block 200.

The bite projection 215 is formed in a bottom of the bite groove 214 and prevents the bite block 200 from slipping relative to the upper or lower teeth of the patient.

The usage of the LED oral illuminator using the bite block is briefly described below according to an embodiment of the present invention.

In advance, the battery 330 is charged. In case that a sign that the battery 330 is charged completely is displayed in the display unit, the frame 300 and the bite block 200 are coupled to open the upper and lower teeth of the patient and up and down.

Next, the light source control switch is manipulated to turn on the LED light source being illuminant. Power supply is provided from the battery 330 to each of the LED light source 340. Each of the LED light source 340 is turned on to illuminate the mouth cavity of the patient. A doctor practices a lesion in a mouth of a patient.

Then, when the doctor performed treatment completely, he/she separates the bite block 200 and the frame 300 from teeth of the patients. He /she separates the bite block 200 from the frame 300 and washes the bite block in high temperature.

After that, the bite block 200 is exposed at steam with high temperature for a predetermined time and sterilized so as to keep the bite block 200 sanitarily.

The bite block 200 is attachable to the frame 300. The battery 330 or the LED light source 340 do not formed in the bite block 200. The battery 330 and the LED light source 340 are included in the frame 300. From these structures, the damage and life shortage of the battery 330 and the LED light source 340 due to washing or sterilizing can be prevented.

Although the present invention has been described herein with reference to the foregoing embodiments and the accompanying drawings, the scope of the present invention is defined by the claims that follow. Accordingly, those skilled in the art will appreciate that various substitutions, modifications and changes are possible, without departing from the spirit of the present invention as disclosed in the accompanying claims. It is to be understood that such substitutions, modifications and changes are within the scope of the present invention.

### [Industrial Applicability]

The LED oral illuminator according to an embodiment of the present invention illuminates a mouth cavity of a patient using an LED light source located in a bite block. The LED intraoral device according to the present invention can illuminate mouth cavity, in particularly, lingual sides of upper and lower teeth, the other ends of the upper and lower teeth facing moral biting the bite block, throat, subglossal and so forth as a whole to be used widely in dental treatment field.

## Claims

1. An LED oral illuminator using a bite block comprising:
a frame being bent to be U-shaped as a whole; and
a bite block coupled to one end of the frame,
wherein the frame comprises:
an LED light source mounted on one end of the frame; and
a light source control switch for controlling turn on/off of the LED light source.

2. The LED oral illuminator using a bite block according to claim 1, further comprising a battery mounted on the other end of the frame.

3. The LED oral illuminator using a bite block according to claim 2, wherein the LED light source comprises a plurality of LED lamps.

4. The LED oral illuminator using a bite block according to claim 1, wherein the bit block comprises:
a block body bitten between upper and lower teeth in one side of a mouth of a patient to open the mouth of the patient; and
a light source insert hole formed in one side of the block body so that the LED light source us inserted into the light source insert hole.

5. The LED oral illuminator using a bite block according to claim 4, wherein a bite groove bitten between the upper and lower teeth is formed at upper and lower portions of the block body.

6. The LED oral illuminator using a bite block according to claim 5, wherein the bite block is made of transparent material so as to transmit the light source into the mouth.

7. The LED oral illuminator using a bite block according to any one of claims 1 through 3, wherein the battery is chargeable, and the frame further includes an electrical charging terminal for charging the battery.

8. The LED oral illuminator using a bite block according to claim 1, wherein the frame further includes brightness controller for adjusting brightness of the LED light source.

9. The LED oral illuminator using a bite block according to claim 1, wherein the LED light source further includes an UV LED lamp for emitting ultraviolet rays.
